# EUROPEAN PATENT SPECIFICATION

(11) **EP 0 639 380 B1**
(45) Date of publication and mention of the grant of the patent: **29.03.2000**
(21) Application number: 94106929.6
(22) Date of filing: 04.05.1994
(51) Int. Cl.: A61K 47/48, A61K 31/335

(54) **Cyclodextrin inclusion product of taxol, process for producing the same, and its use**
Cyclodextrin Einschluskomplexe mit Taxol, ein Verfahren zu ihrer Herstellung und ihre Anwendung
Produits d'inclusion du taxol dans la cyclodextrine, leur préparation et application

(30) Priority: 19.08.1993 JP 22499993
(43) Date of publication of application: 22.02.1995
(73) Proprietor: Ensuiko Sugar Refining Company, Limited, Kanagawa-ken,230 (JP)
(72) Inventor: Takahashi, Hideki, c/o Ensuiko Sugar Ref.Co.,Ltd., Yokohama-shi, Kanagawa-ken (JP); Saito, Kyoko, c/o Ensuiko Sugar Ref.Co.,Ltd., Yokohama-shi, Kanagawa-ken (JP); Mikuni, Katsuhiko, c/o Ensuiko Sugar Ref.Co.,Ltd., Yokohama-shi, Kanagawa-ken (JP); Kuwahara, Nobuhiro, c/o Ensuiko Sugar Ref.Co.,Ltd., Yokohama-shi, Kanagawa-ken (JP); Hamada, Hiroki, c/o Okayamarika-daigaku, Okayama-shi, Okayama-ken (JP)
(74) Representative: Türk - Gille - Hrabal - Struck

(56) References cited:
- Week 9410, Derwent Publications Ltd., London, GB; AN 94-074757 & AU-B-645 927 (ENSUIKO SUGAR REFINING CO LTD)

## Description

### 1. Field of the Invention

The present invention relates to the improvement of the solubility of taxol in water. More particularly, it relates to the improvement of the solubility of taxol in aqueous phase through conversion into an inclusion product included in cyclodextrin (hereinafter referred to as CD).

### 2. Prior Art

Taxol is a compound extracted from the bark of Taxus brevifolia, a type of yew tree which grows in North America. The compound has a function of suppressing the division of cancer cells. It has been observed that the compound can be particularly effective when administered to patients suffering from ovarian cancer. Much attention has therefore been paid to the substance as a novel, effective anti-cancer drug. However, taxol, when administered to patients, was hardly adsorbed due to its insolubility in water.

Taxol has been only recently discovered, and little has been reported so far on its solubility, except the disclosure in the specification of Japanese Patent Application No. Hei4-339, 495 (339,495/1992), in which the solubility of taxol is improved by allowing the compound to react in a mixture of an organic solvent and water with α -CD, β -CD, γ -CD or a substituted α -, β - or γ -CD at least one of the hydroxyl groups of which is substituted through an ether bond with at least one glucosyl group, maltosyl group or maltooligosaccharide residue.

In the above method, organic solvents are used for the preparation of CD inclusion products. The use of organic solvents, however, is disadvantageous from the point of view of cost, makes the operation complicated and allows for the possibility of the contamination of organic solvents.

### SUMMARY OF THE INVENTION

It is an object of the invention to provide a novel inclusion product of taxol having an improved solubility in water.

It is another object of the invention to provide a method for producing a CD inclusion product of taxol in a convenient, economically advantageous manner, without the use of organic solvents.

Other objects of the invention will become apparent from the following description.

The invention provides:
(1) An inclusion product of taxol included in a substituted α -, β - or γ -CD, one or more hydroxyl groups of which are substituted through an ether bond with at least one member selected from a group consisting of methyl, hydroxyethyl and hydroxypropyl groups, provided that the substituted β-cyclodextrin is not haptakis-2,6-O-dimethyl-β-cyclodextrin, randomly methylated β-cyclodextrin or 2-hydroxypropyl-β-cyclodextrin.
(2) A method for producing an inclusion product of taxol included in CD, which method comprises reacting taxol with α -CD, or a substituted α -, β - or γ -CD, one or more hydroxyl groups of said substituted CD being substituted through an ether bond with at least one member selected from a group consisting of glucosyl group, maltosyl group, maltooligosaccharide residue, methyl group, hydroxyethyl group and hydroxypropyl group, and said reaction being conducted in an aqueous system while stirring or shaking, provided that the substituted β-cyclodextrin is not haptakis-2,6-O-dimethyl-β-cyclodextrin, randomly methylated β-cyclodextrin or 2-hydroxypropyl-β-cyclodextrin.
(3) A method for producing an inclusion product of taxol included in CD, which method comprises reacting taxol with a substituted α -, β - or γ -CD, one or more hydroxyl groups of said substituted CD being substituted through an ether bond with at least one member selected from a group consisting of methyl, hydroxyethyl and hydroxypropyl groups, and said reaction being conducted in an organic solvent-water system while stirring or shaking, provided that the substituted β-cyclodextrin is not heptakis-2,6-O-dimethyl-β-cyclodextrin, randomly methylated β-cyclodextrin or 2-hydroxypropyl-β-cyclodextrin.
(4) A method for improving the solubility of taxol in water, which method comprises reacting taxol with α -CD, or a substituted α -, β - or γ -CD, one or more hydroxyl groups of said substituted CD being substituted through an ether bond with at least one member selected from the group consisting of glucosyl group, maltosyl group, maltooligosaccharide residue, methyl group, hydroxyethyl group and hydroxypropyl group, and said reaction being conducted in an aqueous system while stirring or shaking, provided that the substituted β-cyclodextrin is not heptakis-2,6-O-dimethyl-β-cyclodextrin, randomly methylated β-cyclodextrin or 2-hydroxypropyl-β-cyclodextrin. and
(5) A method for improving the solubility of taxol in water, which method comprises reacting taxol with a substituted α -, β - or γ -CD, one or more hydroxyl groups of said substituted CD being substituted through an ether bond with at least one member selected from the group consisting of methyl, hydroxyethyl and hydroxypropyl groups, and said reaction being conducted in an organic solvent-water system while stirring or shaking provided that the substituted β-cyclodextrin is not heptakis-2,6-O-dimethyl-β-cyclodextrin, randomly methylated β-cyclodextrin or 2-hydroxypropyl-β-cyclodextrin.

### BRIEF DESCRIPTION OF THE DRAWING

Fig. 1 is a graph showing the change with the lapse of time of the amount of taxol dissolved into an aqueous 0.1 M solution of 2, 6-di-O-methyl-β-CD (DM-β-CD).
Fig. 2 is a spectrum of ¹H-NMR analysis for taxol-DM-β-CD complex powder dissolved in D₂O.
Fig. 3 is a spectrum of ¹H-NMR analysis for taxol dissolved in D₂O.
Fig. 4 is a spectrum of ¹H-NMR analysis for taxol-DM-β-CD complex powder dissolved in d₆₋DMSO.
Fig. 5 is a spectrum of ¹H-NMR analysis for taxol dissolved in d₆₋DMSO.
Fig. 6 is a spectrum of ¹H-NMR analysis for DM-β-CD dissolved in d ₆₋DMSO.
Fig. 7 is a spectrum of ¹³ C-NMR analysis for taxol-DM-β-CD complex powder dissolved in d₆₋DMSO.
Fig. 8 is a spectrum of ¹³C-NMR analysis for DM-β-CD dissolved in d₆₋DMSO.

### DETAILED DESCRIPTION OF THE INVENTION

By the term CD is meant a compound consisting of a ring of 6 to 8 D-glucoses linked by α -1,4 bonds; one consisting of 6 D-glucoses is referred to as α -CD, one consisting of 7 D-glucoses as β -CD, and one consisting of 8 D-glucoses as γ -CD.

In the invention is used these α -, -CD per se, or a substituted α -, β - or γ -CD, at least one of the hydroxyl groups of which is substituted with at least one functional group through an ether bond. Examples of usable substituted CDs include those substituted with such functional groups as saccharide moieties (e. g. , glucosyl, maltosyl, maltooligo-saccharide), alkyl groups (e.g., methyl, ethyl), hydroxyalkyl groups (e. g., hydroxyethyl, hydroxypropyl), provided that the substituted β-cyclodextrin is not heptakis-2,6-O-dimethyl-β-cyclodextrin, randomly methylated β-cyclodextrin or 2-hydroxypropyl-β-cyclodextrin, and the like.

These CDs can be used either alone or in combination of two or more.

In order to obtain a CD inclusion product of taxol by a reaction in an aqueous system, the CD is dissolved into water, and taxol is then added there to, followed by vigorous stirring or shaking. The concentration of the CD can be from 0. 0001 to 200% by weight, preferably from 1 to 50% by weight, based on the weight of water.

The CD is admixed in an amount of 1 to 100, 000, 000 times the mole amount of taxol preferably 100 to 100, 000 times the mole amount of taxol. The stirring or shaking is conducted as vigorously as possible for a period of from a few minutes to several tens of minutes. The reaction is carried out at a temperature of 0 to 60°C preferably 15 to 40°C.

By the above reaction, taxol is included within the CD. When taxol is admixed at a high ratio, part of the compound may remain undissolved. In such a case, the undissolved compound can be removed from the reaction mixture by means of filtration.

The thus-obtained aqueous solution of the CD inclusion product of taxol can be dried, if necessary, to obtain powders of the inclusion product. The resulting CD inclusion product of taxol has a markedly improved solubility in water.

In order to obtain a CD inclusion product of taxol by a reaction in an organic solvent-water system (namely, a mixture of an organic solvent and water), taxol is at first dissolved into an organic solvent.

Examples of organic solvents usable for the dissolution include ethyl acetate, methanol, ethanol, acetone, acetonitrile tetrahydrofuran, dimethylsulfoxide, and the like. Acetonitrile and methanol are particularly preferred. There is no particular restriction on the amount of organic solvents to be used, provided that taxol can be dissolved.

On the other hand, the CD is dissolved into water to prepare an aqueous solution containing the CD in an amount of 1 to 100, 000, 000 times, preferably 100 to 100, 000 times the mole amount of the taxol, and the solution is added to the above taxol solution. Alternatively, the CD is added directly to a taxol solution. Upon the addition, the taxol solution is stirred, preferably in a vigorous manner using a stirrer or the like. There is no particular restriction on the temperature at which the inclusion reaction is carried out, and the reaction proceeds to a sufficient degree at room temperature. The reaction time can be in the range of from a few minutes to several hours.

By the above reaction, taxol is included within the CD.

The resulting solution can be dried, if desired, to obtain a stable CD inclusion product of taxol. The thus obtained product has a high solubility in water.

The thus-obtained CD inclusion product of taxol, when administered to a patient, e. g. , by means of intravenous injection or oral administration, can be effectively transported into the diseased part of the patient at a smaller dosage. Because of this, the physiological activities of taxol can be used in an effective manner.

The CD inclusion product of taxol obtainable by the invention can be used in various forms. For example, it can be prepared as an injection drug, or can be used as it is in the form of a powder. For the purpose of convenience, the powder can be shaped into granules or tablets, or can be filled into a capsule. The inclusion product can, of course, be used in the form of a solution.

Inclusion products of taxol according to this invention have a markedly improved solubility in aqueous phase and hence can be delivered in an effective manner to diseased body parts of patients suffering from cancer.

The invention will further be illustrated by examples. It should however be noted that the invention is by no means limited by the examples.

### Example 1

Into water were dissolved 0.058 g of α -CD, 0.078 g of γ -CD, 0.078 g of 6-mono-O-maltosyl- α -CD (hereinafter referred to as G₂- α -CD), 0.088 g of 6-mono-O-maltosyl-β-CD (hereinafter referred to as G₂-β-CD), 0.097 g of 6-mono-O-maltosyl- γ -CD (hereinafter referred to as G₂- γ -CD), 0.069 g of 2, 6-di-O-methyl- α -CD (hereinafter referred to as DM- α -CD), 0.080 g of 2, 6-di-O-methyl- β -CD (hereinafter referred to as DM-β-CD), and 0. 098 g of hydroxyethyl-β-CD in which, of 21 hydroxyl groups contained in one β -CD molecule, an average of 1.6 groups are substituted through an ether bond with a hydroxyethyl group (hereinafter referred to as HE-β-CD) or 0.090 g of hydroxypropyl-β-CD in which, of 21 hydroxyl groups contained in one β -CD molecule, an average of 0.9 groups are substituted through an ether bond with a hydroxypropyl group (hereinafter referred to as HP-β -CD), and the volume of each solution was adjusted to 10 ml to prepare aqueous CD solutions of a concentration of 6 mM.

To the solutions were added 2 mg each of taxol, and the resulting mixtures were stirred at room temperature (20 to 25°C) at 5,000 r.p.m. As a control, 2 mg of taxol were added to 10 ml of water, and the resulting mixture was stirred at room temperature (20 to 25°C) at 5,000 r.p.m. After 70 minutes of stirring, the solutions were passed through a filter of a pore diameter of 0. 45µm, and the concentration of taxol contained in the filtrates was determined by highperformance liquid chromatography (HPLC). The results obtained are shown in Table 1.

**Table 1**

| | Solubility of Taxol (µ g/ml) |
|---|---|
| CD not added (Control) | 0 |
| α -CD | 0.3 |
| γ -CD | 0.3 |
| G₂-α-CD | 0.3 |
| G₂-β-CD | 0.5 |
| G₂-γ-CD | 0.4 |
| DM-α-CD | 2.8 |
| DM-β-CD | 47.1 |
| HE-β-CD | 0.5 |
| HP-β-CD | 1.1 |

### Example 2

Into water were dissolved 1.296 g of G₂-α-CD, 1.458 g of G₂-β-CD, 1.620 g of G₂-γ-CD, 1.331 g of DM-β-CD, 1.429 g of 2,3, 6-tri-O-methyl-β-CD (hereinafter referred to as TM-β-CD), and 1.629 g of HE-β-CD or 1.501 g of HP-β-CD, and the volume of each solution was adjusted to 10 ml to prepare aqueous CD solutions of a concentration of 100 mM. Then, 20 mg of taxol were added to the aqueous DM-β-CD solution, 2 mgof taxol to the aqueous HP-β-CD solution, and 1 mg each of taxol to the other aqueous CD solutions, and the resulting mixtures were stirred at room temperature (20 to 25°C) at 5,000 r.p.m. As a control, 1 mg of taxol was added to 10 ml of water, and the resulting mixture was stirred at room temperature (20 to 25°C) at 5,000 r.p.m. After 70 minutes of stirring, the solutions were passed through a filter with a pore diameter of 0.45µm, and the concentration of taxol contained in the filtrates was determined. The results obtained are shown in Table 2.

**Table 2**

| | Solubilty of Taxol ( µ g/ml) |
|---|---|
| CD not added (Control) | 0.4 |
| G₂-α-CD | 1.6 |
| G₂-β-CD | 23.0 |
| G₂-γ-CD | 1.5 |
| DM-β-CD | 1236 |
| TM-β-CD | 28.0 |
| HE-β-CD | 22.2 |
| HP-β-CD | 68.9 |

### Example 3

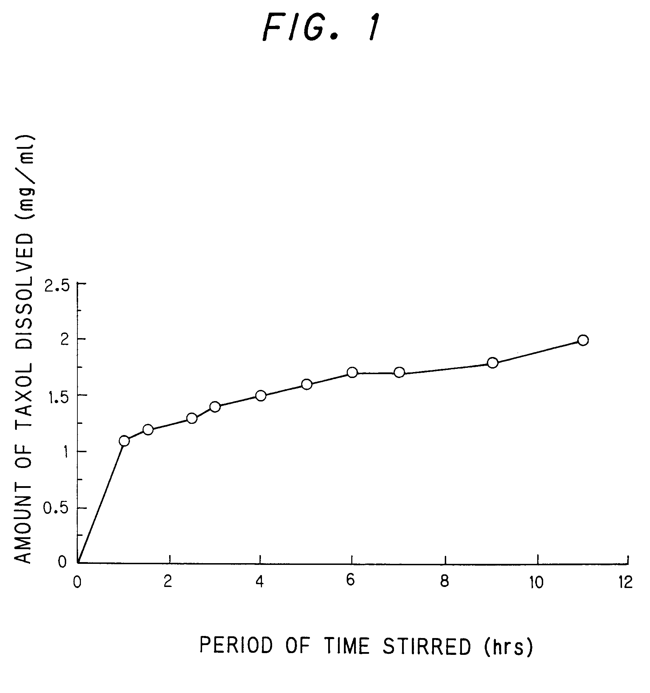

Into water was dissolved 1.331 g of DM-β-CD, and the volume of the solution was adjusted to 10 ml to prepare an aqueous CD solution of a concentration of 100 mM. To this were added 20 mg of taxol, and the resulting mixture was stirred at room temperature (20 to 25 °C) at 5,000 r.p.m. for a period of 11 hours. While stirring, samples of the mixture were taken at appropriate intervals and passed through a filter with a pore size of 0. 45µm, and the concentration of taxol contained in the filtrates was determined. The results obtained are shown in Fig. 1.

### Example 4

Into 70 ml of a mixture of methanol and water (volume ratio, 1:1), the same volume of a mixture of acetonitrile and water (volume ratio, 1: 1), the same volume of a mixture of acetone and water (volume ratio, 1: 1) and the same volume of mixture of tetrahydrofuran and water (volume ratio, 1:1) were added 5 mg each of taxol. To the resulting taxol solutions were added 500 mg each of DM-β-CD, and the resulting mixtures were stirred vigorously by a stirrer at a temperature of 25°C. After two hours of stirring, 100 ml each of water were added thereto, and the resulting solutions were freeze-dried, so as to obtain powders by completely removing the water and the organic solvents. Then, 8 mg each of the thus-obtained powders were dissolved into 1 ml of water while stirring, and the resulting solutions were passed through a filter with a pore size of 0. 45µm, and the concentration of DM-β-CD and of the taxol contained in the filtrates was determined by HPLC. Table 3 shows the solubility of taxol at a DM-β-CD concentration of 6 mM.

**Table 3**

| Organic Solvent Used | Solubility of Taxol (µg/ml) |
|---|---|
| Methanol | 63.2 |
| Acetonitrile | 78.6 |
| Acetone | 43.9 |
| Tetrahydrofuran | 10.3 |

### Example 5

Two taxol solutions were prepared by dissolving 5 mg each of taxol into 100 ml each of mixtures of methanol and water (volume ratio, 1:1). To these solutions were added 50 mg of HE-β-CD or HP-β-CD, and the resulting mixtures were vigorously stirred using a stirrer at 25°C. After 2 hours of stirring, 300 ml of water were added thereto and the resulting solutions were subjected to freeze-drying, so as to obtain powders by completely removing the water and the organic solvent.

Parts of the thus-obtained powders (9.8 mg of HE-β-CD and 9.0 mg of HP-β-CD) were dissolved into 1 ml each of water. After being stirred, the resulting mixtures were passed through a filter with a pore size of 0. 45 µ m , and the concentration of taxol contained in the filtrates was deter mined. The results obtained are shown in Table 4.

**Table 4**

| CD Used | Solubility of Taxol (µ g/ml) |
|---|---|
| HE-β-CD | 1.3 |
| HP-β-CD | 6.1 |

### Example 6

13.31 g of DM-β-CD was dissolved into water and the volume of the solution was adjusted to 100 ml to prepare aqueous CD solution of a concentration of 0.1M.

To the solution was added 1.2 g of taxol, and the resulting mixtures was stirred by a stirrer at 25°C for 1 hour. Subsequently, the mixture was filtered with a membrane filter having a pore size of 0. 45 µ m to remove the undissolved taxol, thereby a transparent solution was obtained. The resulting solution was freeze-dried to obtain taxol-DM- β-CD complex in powder form.

0.05 g of taxol-DM-β-CD complex powder was dissolved into water to make 10 ml of solution. Then, 20 µ l of the solution was subjected to HPLC analysis under the conditions as: column: Crest Pak C18S (JASCO CO. ,Japan), column temperature: room temperature, eluent: methanol/water=6 5/35 (by volume), flow rate: 1.0 ml/min., detector: UV detector, and measured wave length: UV 230 nm. As the standard solutions of taxol, each 50 µ g/ml, 100 µ g/ml, 150 µ g/ml, and 200 µ g/ml of taxol solution dissolved in methanol/water (65/35 by volume) was prepared, and each 20 µ l of these solutions was analyzed under the same analytical conditions as above to obtain a calibration curve. According to the absolute calibration curve method, taxol content in the taxol-DM-β-CD complex powder was determined to be 10.7 mg/g-powder.

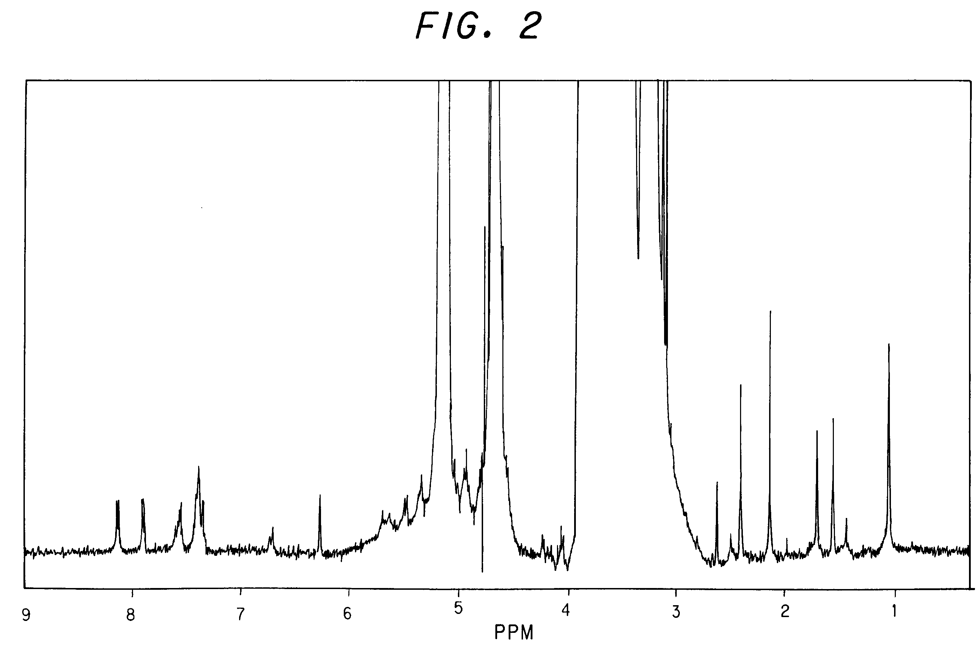

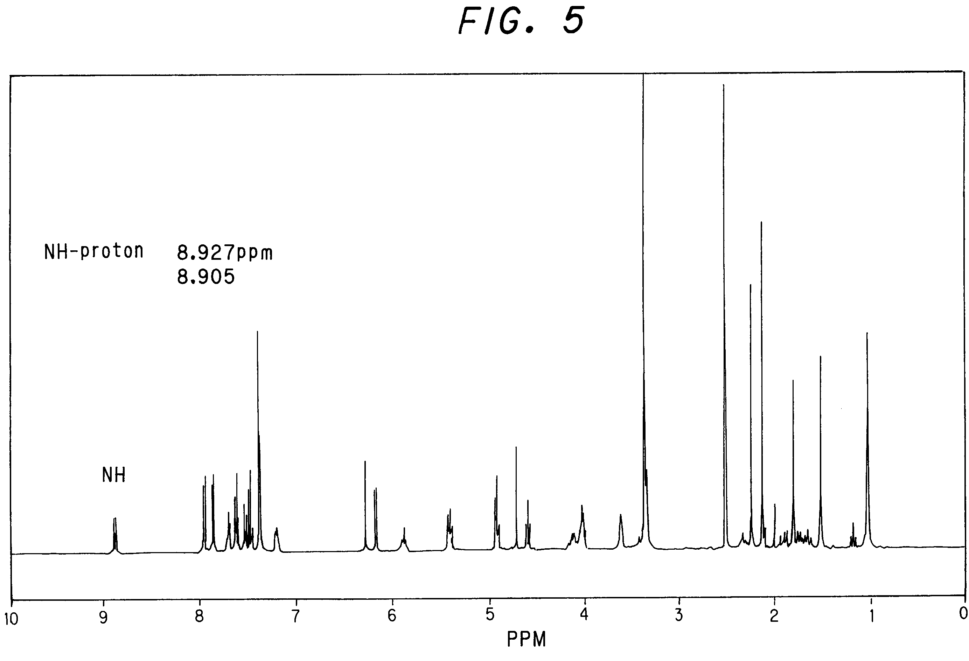

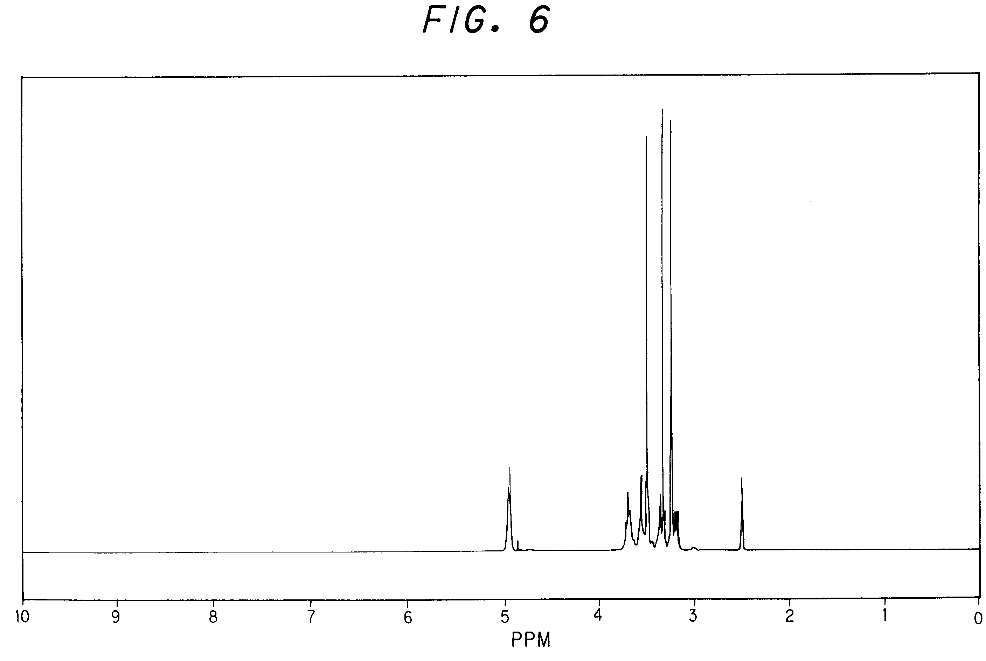

Taxol-DM-β-CD complex powder, taxol and DM-β-CD were dissolved into D₂O or d₆-DMSO. The resultant solution was subjected to ¹H-NMR (400 MHz) or ¹⁸C-NMR (100 MHz) analysis with the use of JNM-GSX 400 spectrometer (manufactured by JEOL, Ltd.). The results were shown Fig. 2 to 8.

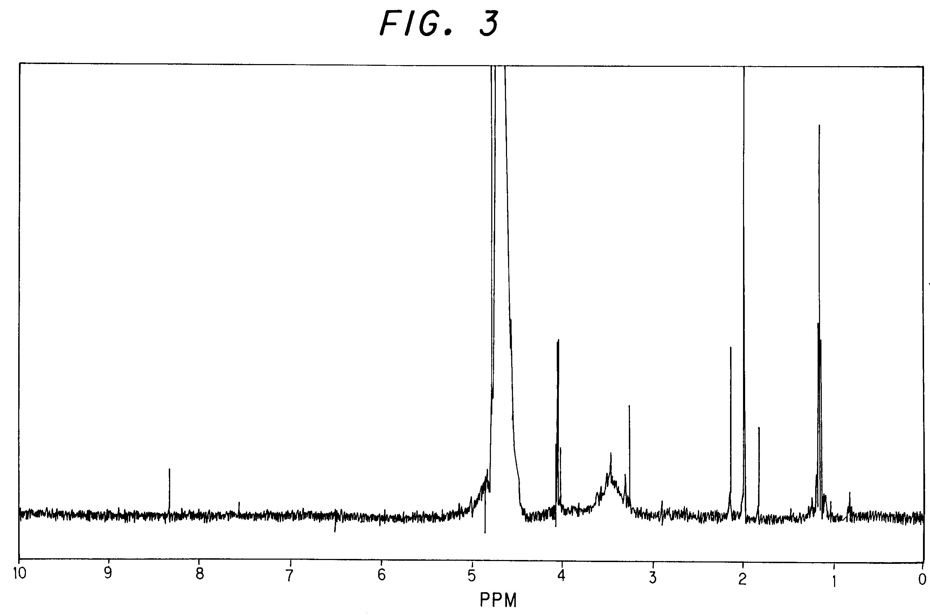

Fig. 2 and 3 are spectra of ¹H-NMR analysis for taxol-DM-β-CD complex powder and taxol dissolved in D₂O, respectively. A spectrum of taxol is clear in Fig. 2, but not in Fig. 3.

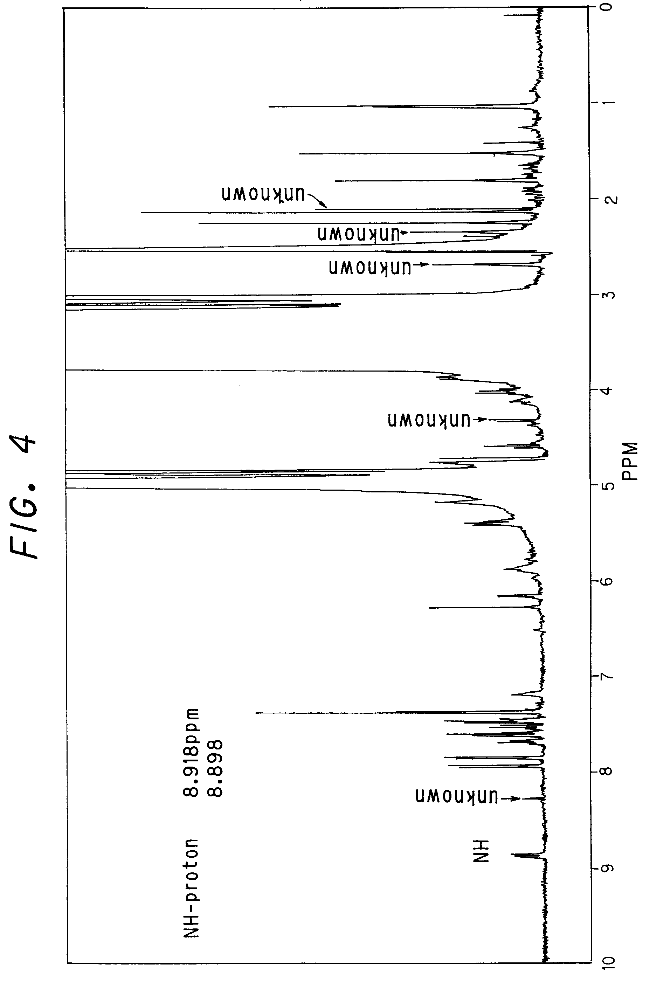

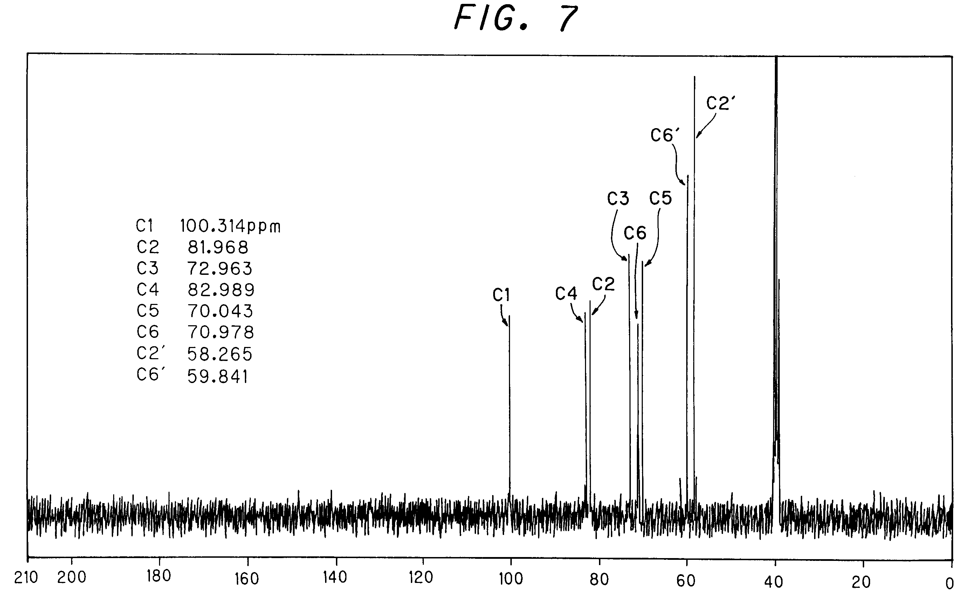

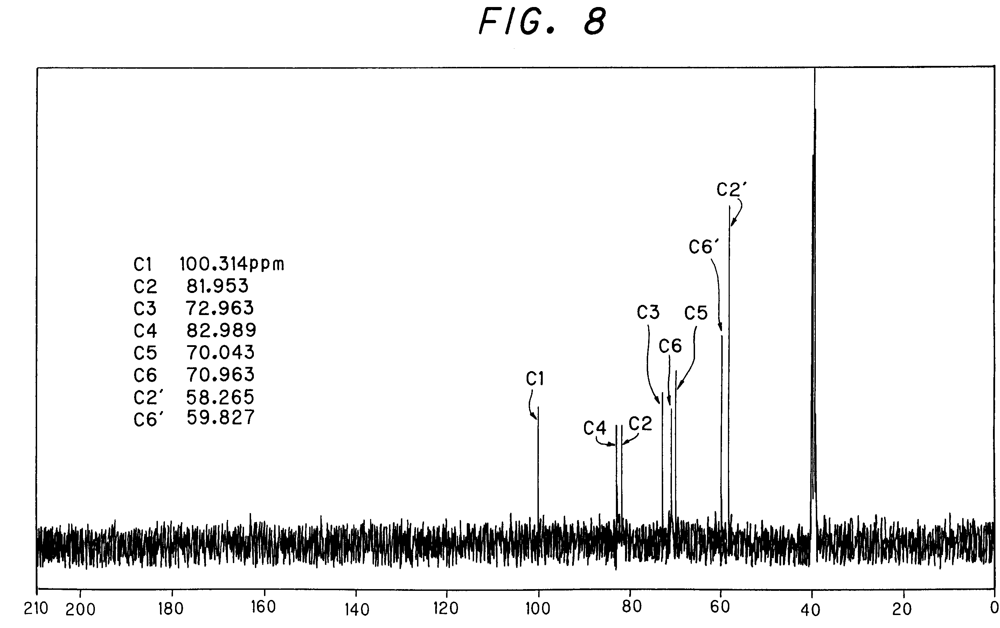

Fig. 4, 5 and 6 are spectra of ¹H-NMR analysis for taxol-DM-β-CD complex powder, taxol and DM-β-CD each dissolved in d₆₋DMSO, respectively. By comparing each chemical shift of taxol in the taxol-DM-β-CD complex and free taxol it was found that NH proton among signals of taxol in the taxol-DM-β-CD complex was shifted upfield, and in the spectrum of taxol in the taxol-DM-β-CD complex there were existed signals different from these of the spectrum of free taxol. Fig. 7 and 8 are spectra of ¹³C-NMR analysis for taxol-DM-β-CD complex powder and for DM-β-CD dissolved in d₆₋DMSO, respectively. By comparing each chemical shift of DM-β-CD in the taxol-DM-β-CD complex and free DM-β-CD, it was found that C-2, C-6 and C-6' (carbon of methoxyl group) in the glucose unit of CD in the taxol-DM- β -CD complex were shifted downfield.

By the above results, it was found that the solubility of taxol to water was improved by the existence of CD, and thus the interaction between taxol and DM-β-CD was suggested.

## Claims

1. An inclusion product of taxol included in a substituted α-, β - or γ -cyclodextrin, one or more hydroxyl groups of which are substituted through an ether bond with at least one member selected from the group consisting of methyl, hydroxyethyl and hydroxypropyl groups, provided that the substituted β-cyclodextrin is not heptakis-2,6-O-dimethyl-β-cyclodextrin, randomly methylated β-cyclodextrin or 2-hydroxypropyl-β-cyclodextrin.

2. A method for producing an inclusion product of taxol included in cyclodextrin, which method comprises reacting taxol with α - cyclodextrin, or a substituted α -, β - or γ -clodextrin, one or more hydroxyl groups of said substituted cyclodextrin being substituted through an ether bond with at least one member selected from the group consisting of glucosyl group, maltosyl group, maltooligosaccharide residue, methyl group, hydroxyethyl group and hydroxypropyl group, and said reaction being conducted in an aqueous system while stirring or shaking, provided that the substituted β-cyclodextrin is not heptakis-2,6-O-dimethyl-β-cyclodextrin, randomly methylated β-cyclodextrin or 2-hydroxypropyl-β-cyclodextrin.

3. A method for producing an inclusion product of taxol included in cyclodextrin, which method comprises reacting taxol with a substituted α-, β- or γ -cyclodextrin, one or more hydroxyl groups of said substituted cyclodextrin being substituted through an ether bond with at least one member selected from the group consisting of methyl, hydroxyethyl and hydroxypropyl groups, and said reaction being conducted in an organic solvent-water system while stirring or shaking, provided that the substituted β-cyclodextrin is not heptakis-2,6-O-dimethyl-β-cyclodextrin, randomly methylated β-cyclodextrin or 2-hydroxypropyl-β-cyclodextrin.

4. A method for improving the solubility of taxol in water, which method comprises reacting taxol with α-cyclodextrin, or a substituted α-, β - or γ -cyclodextrin, one or more hydroxyl groups of said substituted cyclodextrin being substituted through an ether bond with at least one member selected from the group consisting of glucosyl group, maltosyl group, maltooligosaccharide residue, methyl group, hydroxyethyl group and hydroxypropyl group, and said reaction being conducted in an aqueous system while stirring or shaking, provided that the substituted β-cyclodextrin is not heptakis-2,6-O-dimethyl-β-cyclodextrin, randomly methylated β-cyclodextrin or 2-hydroxypropyl-β-cyclodextrin.

5. A method for improving the solubility of taxol in water, which method comprises reacting taxol with a substituted α-, β - or γ - cyclodextrin, one or more hydroxyl groups of said substituted cyclodextrin being substituted through an ether bond with at least one member selected from the group consisting of methyl, hydroxyethyl and hydroxypropyl groups, and said reaction being conducted in an organic solvent-water system while stirring or shaking, provided that the substituted β-cyclodextrin is not heptakis-2,6-O-dimethyl-β-cyclodextrin, randomly methylated β-cyclodextrin or 2-hydroxypropyl-β-cyclodextrin.

## Patentansprüche

1. Einschlußprodukt von Taxol, das eingeschlossen ist in einem substituierten α-, β- oder γ-Cyclodextrin, in dem ein oder mehrere Hydroxylgruppen über eine Etherbindung mit mindestens einem Bestandteil, ausgewählt aus der Gruppe, die aus Methyl-, Hydroxyethyl- und Hydroxypropyl-Gruppen besteht, substituiert sind, mit der Maßgabe, daß das substituierte β-Cyclodextrin nicht Heptakis-2,6-O-dimethyl-β-cyclodextrin, statistisch methyliertes β-Cyclodextrin oder 2-Hydroxypropyl-β-cyclodextrin ist.

2. Verfahren zur Herstellung eines Einschlußproduktes von Taxol, das in Cyclodextrin eingeschlossen ist, das umfaßt: Umsetzen von Taxol mit α-Cyclodextrin oder einem substituierten α-, β- oder γ-Cyclodextrin, in dem ein oder mehrere Hydroxylgruppen des genannten substituierten Cyclodextrins über eine Etherbindung mit mindestens einem Bestandteil substituiert sind, der aus der Gruppe ausgewählt wird, die aus einer Glucosylgruppe, einer Maltosylgruppe, einem Maltooligosaccharidrest, einer Methylgruppe, einer Hydroxyethylgruppe und einer Hydroxypropylgruppe besteht, und worin die genannte Reaktion in einem wäßrigen System unter Rühren oder Schütteln durchgeführt wird, mit der Maßgabe, daß das substituierte β-Cyclodextrin nicht Heptakis-2,6-O-dimethyl-β-cyclodextrin, statistisch methyliertes β-Cyclodextrin oder 2-Hydroxypropyl-β-cyclodextrin ist.

3. Verfahren zur Herstellung eines Einschlußproduktes von Taxol, das in Cyclodextrin eingeschlossen ist, das umfaßt: Umsetzen von Taxol mit einem substituierten α-, β- oder γ-Cyclodextrin, worin ein oder mehrere Hydroxylgruppen des genannten substituierten Cyclodextrins über eine Etherbindung mit mindestens einem Bestandteil substituiert sind, der ausgewählt wird aus der Gruppe, die aus Methyl-, Hydroxyethyl- und Hydroxypropylgruppen besteht, und worin die genannte Reaktion in einem organischen Lösungsmittel-Wasser-System unter Rühren oder Schütteln durchgeführt wird, mit der Maßgabe, daß das substituierte β-Cyclodextrin nicht Heptakis-2,6-O-dimethyl-β-cyclodextrin, statistisch methyliertes β-Cyclodextrin oder 2-Hydroxypropyl-β-cyclodextrin ist.

4. Verfahren zur Verbesserung der Löslichkeit von Taxol in Wasser, das umfaßt: Umsetzen von Taxol mit α-Cyclodextrin oder einem substituierten α-, β- oder γ-Cyclodextrin, in dem ein oder mehrere Hydroxylgruppen des genannten substituierten Cyclodextrins über eine Etherbindung mit mindestens einem Bestandteil substituiert sind, der aus der Gruppe ausgewählt wird die aus einer Glukosylgruppe, einer Maltosylgruppe, einem Maltooligosaccharidrest, einer Methylgruppe, einer Hydroxyethylgruppe und einer Hydroxypropylgruppe besteht, und worin die genannte Reaktion in einem wäßrigen System unter Rühren oder Schütteln durchgeführt wird, mit der Maßgabe, daß das substituierte β-Cyclodextrin nicht Heptakis-2,6-O-dimethyl-β-cyclodextrin, statistisch methyliertes β-Cyclodextrin oder 2-Hydroxypropyl-β-cyclodextrin ist.

5. Verfahren zur Verbesserung der Löslichkeit von Taxol in Wasser, das umfaßt: Umsetzen von Taxol mit einem substituierten α-, β- oder γ-Cyclodextrin, worin ein oder mehrere Hydroxylgruppen des genannten substituierten Cyclodextrins über eine Etherbindung mit mindestens einem Bestandteil substituiert sind, der aus der Gruppe ausgewählt wird, die aus einer Methyl-, einer Hydroxyethyl- und einer Hydroxypropylgruppe besteht, wobei die genannte Reaktion in einem organischen Lösungsmittel-Wasser-System unter Rühren oder Schütteln durchgeführt wird, mit der Maßgabe, daß das substituierte β-Cyclodextrin nicht Heptakis-2,6-O-dimethyl-β-cyclodextrin, statistisch methyliertes β-Cyclodextrin oder 2-Hydroxypropyl-β-cyclodextrin ist.

## Revendications

1. Produit d'inclusion du taxol inclus dans une α-, β-ou γ-cyclodextrine substituée, dont un ou plusieurs groupes hydroxyle sont substitués par l'intermédiaire d'une liaison éther par au moins un membre du groupe comprenant les groupes méthyle, hydroxyéthyle et hydroxypropyle, à condition que la β-cyclodextrine substituée ne soit pas l'heptakis-2,6-O-diméthyl-β-cyclodextrine, la β-cyclodextrine méthylée de façon aléatoire ou la 2-hydroxypropyl-β-cyclodextrine.

2. Procédé pour préparer un produit d'inclusion du taxol inclus dans une cyclodextrine, lequel procédé comprend la mise en réaction du taxol avec une α-cyclodextrine, ou une α-, β- ou γ-cyclodextrine substituée, un ou plusieurs groupes hydroxyle de ladite cyclodextrine substituée étant substitués par l'intermédiaire d'une liaison éther par au moins un membre du groupe comprenant un groupe glucosyle, un groupe maltosyle, un résidu de malto-oligosaccharide, un groupe méthyle, un groupe hydroxyéthyle et un groupe hydroxypropyle, et ladite réaction étant effectuée dans un système aqueux sous agitation ou secousses, à condition que la β-cyclodextrine substituée ne soit pas l'heptakis-2,6-O-diméthyl-β-cyclodextrine, la β-cyclodextrine méthylée de façon aléatoire ou la 2-hydroxypropyl-β-cyclodextrine.

3. Procédé pour préparer un produit d'inclusion du taxol inclus dans une cyclodextrine, lequel procédé comprend la mise en réaction du taxol avec une α-, β- ou γ-cyclodextrine substituée, un ou plusieurs groupes hydroxyle de ladite cyclodextrine substituée étant substitués par l'intermédiaire d'une liaison éther par au moins un membre du groupe comprenant les groupes méthyle, hydroxyéthyle et hydroxypropyle, et ladite réaction étant effectuée dans un système à base de solvant organique-eau sous agitation ou secousses, à condition que la β-cyclodextrine substituée ne soit pas l'heptakis-2,6-O-diméthyl-β-cyclodextrine, la β-cyclodextrine méthylée de façon aléatoire ou la 2-hydroxypropyl-β-cyclodextrine.

4. Procédé pour améliorer la solubilité du taxol dans l'eau, lequel procédé comprend la mise en réaction du taxol avec une α-cyclodextrine, ou une α-, β- ou γ-cyclodextrine substituée, un ou plusieurs groupes hydroxyle de ladite cyclodextrine substituée étant substitués par l'intermédiaire d'une liaison éther par au moins un membre du groupe comprenant un groupe glucosyle, un groupe maltosyle, un résidu de malto-oligosaccharide, un groupe méthyle, un groupe hydroxyéthyle et un groupe hydroxypropyle, et ladite réaction étant effectuée dans un système aqueux sous agitation ou secousses, à condition que la β-cyclodextrine substituée ne soit pas l'heptakis-2,6-O-diméthyl-β-cyclodextrine la β-cyclodextrine méthylée de façon aléatoire ou la 2-hydroxypropyl-β-cyclodextrine.

5. Procédé pour améliorer la solubilité du taxol dans l'eau, lequel procédé comprend la mise en réaction du taxol avec une α-, β- ou γ-cyclodextrine substituée, un ou plusieurs groupes hydroxyle de ladite cyclodextrine substituée étant substitués par l'intermédiaire d'une liaison éther par au moins un membre du groupe comprenant les groupes méthyle, hydroxyéthyle et hydroxypropyle, et ladite réaction étant effectuée dans un système à base de solvant organique-eau sous agitation ou secousses, à condition que la β-cyclodextrine substituée ne soit pas l'heptakis-2,6-O-diméthyl-β-cyclodextrine, la β-cyclodextrine méthylée de façon aléatoire ou la 2-hydroxypropyl-β-cyclodextrine.
